# EUROPEAN PATENT SPECIFICATION

(11) **EP 1 726 949 B1**
(45) Date of publication and mention of the grant of the patent: **03.12.2008**
(21) Application number: 06118320.8
(22) Date of filing: 01.10.2003
(51) Int. Cl.: G01N 33/28

(54) **Oil change determination with identification of the lubricating oil in the machine**
Ölwechselbestimmung mit Identifizierung des Schmieröls in der Maschine
Détermination de vidange avec identification de l'huile lubrifiante dans la machine

(30) Priority: 01.10.2002 GB 0222728
(43) Date of publication of application: 29.11.2006
(62) Divisional of application: 03773724.4
(73) Proprietor: SHELL INTERNATIONALE RESEARCH MAATSCHAPPIJ B.V., 2596 HR Den Haag (NL)
(72) Inventor: Sant, Peter, Chester, Cheshire CH2 4NU (GB); Taylor, Robert, Ian, Chester, Cheshire CH2 4NU (GB)
(74) Representative: du Pont, Jeroen

(56) References cited:
- WO-A-01/90539
- US-A- 5 274 335
- US-A- 5 928 954
- US-B1- 6 422 061

## Description

The present invention belongs to the technical field of identifying a lubricating oil in situ in a machine such as an engine. In particular, the method of the present invention makes use of a lubricating oil containing passive markers capable of detection in situ in a machine such as an engine.

Currently the fluids in an engine, for example, the lubricant and fuel, represent one of the very few aspects of an engine about which an engine management chip receives limited, if any, information on. If the wrong lubricant is used in an engine, sub-optimum vehicle performance and even engine failure can result. Therefore, in order to ensure satisfactory and reliable engine operation, the producer of a vehicle (the Original Equipment Manufacturer, OEM) usually recommends the use of specific lubricants. It would be useful if the engine management system was able to identify the lubricant being used in situ so that it can inform the user or adjust engine operation accordingly in order to prevent or diagnose engine problems.

Different types of lubricating oil require different time periods between oil changes. It may be that there is more than one recommended lubricant for an engine, e.g. one product may be of a standard quality having a given time period between oil changes and a second product may be of higher quality and be capable of remaining in the engine for a longer period of time before an oil change is necessary. It would be useful if the engine management system could distinguish between different types of lubricant and be able to use this information to determine when an oil change is necessary and to indicate this to the vehicle user accordingly.

Previous attempts to develop means for identifying lubricating oils have mainly involved incorporating dyes into the oil, for example as described in US-A-5928954 and EP-A-1001003.

For example, US-A-5928954 describes a method for tagging hydrocarbons such as gasoline, diesel fuel, heating oil, lubricating oil or crude petroleum, wherein the hydrocarbon to be tagged is blended with a relatively small amount of a fluorescent dye. The presence of the tagged hydrocarbon is subsequently determined by exciting the dye to fluoresce at wavelengths in the higher portion of the visible spectral region or the lower portion of the near infrared spectral region.

Similarly, US-A-5525516 discloses a method for imparting invisible marking for identification purposes to petroleum hydrocarbons by incorporating one or more near infrared fluorescing compounds (fluorophores) therein. The fluorophores are detected by exposing the marked hydrocarbon compositions to near infrared radiation having a wavelength in the 670-850 nm range and then detecting the emitted fluorescent light via near infrared light detection means.

EP-A-0637743 describes a method for allowing authentication of a bulk liquid and a method for detecting subsequent dilution of a bulk liquid. Said methods are said to involve introducing a known amount of a chemiluminescent marker substance into undiluted bulk liquid. The bulk liquid to be authenticated or analysed for subsequent dilution is then sampled and said samples are exposed to the conditions required to trigger a chemiluminescent reaction. If a reaction occurs, authenticity is thereby confirmed. Comparison of the characteristics of any reaction with those characteristics corresponding to an undiluted bulk liquid allow determination of whether or not dilution of the bulk liquid has occurred.

US-A-5942444 describes a method of marking a product for identification in which a marker, composed of a print molecule, print molecule analogue, or molecularly imprinted molecule, is added to the product and is subsequently measured in a specific binding assay. It is indicated in US-A-5942444 that producing a sample of a product to assay for a marker will comprise one or more steps selected from extraction of a marker compound from the product; dilution of the product with an aqueous or an organic solvent; filtration; evaporation; precipitation; and solid phase extraction of the marker compound, e.g. purification of the marker compound using an ion exchange resin, chromatography (e.g. using silica), or molecularly imprinted solid phase extraction (MISPE) chromatography.

US-A-5429952 discloses a method of marking a product and subsequently detecting the marker in the product as a means of identifying the product, comprising the steps of associating a hapten as a marker with the product, wherein the hapten is non-deleterious to the product, inert with respect to the product, and not already associated with the product; and detecting the hapten in the product at a later point in time as a means of identifying the product by specifically binding the hapten to a complementary binding member. Whilst it is indicated that oil-based products such as lubricating oils, gasoline, diesel and liquefied petroleum products may be marked and analysed by the method of US-A-5429952, immunoassay requires the marker to be brought into aqueous solution. In the case of a marked oil-based product, this is said to require solvent extraction. Indeed, in this regard, Example 3 of US-A-5429952 illustrates the assay of a marked lubricating oil comprising extraction of the m-phenoxybenzoic acid marker therein with 5 volumes of hexane and 1 volume of 20 % acetonitrile in 0.05 M Tris/HCl pH 7.5 prior to immunoassay.

US-A-5776713, which is a continuation-in-part of US-A-5429952, describes a similar method to that described in US-A-5429952, wherein the marker is a hapten which is covalently bound to a pre-formed polymeric compound.

WO-A-96/00271 describes the use of carbonyl compounds for marking hydrocarbons such as motor fuels, heating oils and engine oils. However, detection of said compounds requires treatment of a sample of said hydrocarbons with a solution of an iron (III) salt in aqueous alcohol or aqueous acetone. For example, it is indicated that testing of hydrocarbons which are in a liquid state of aggregation at standard temperature and pressure is usually carried out by vigorously shaking about 20 ml of the marker-containing hydrocarbon with about 2 ml of reagent solution for about 15 to 20 seconds. Upon phase separation, the lower aqueous phase will have undergone a colour change.

The problem with the above prior methods is that in order to identify a lubricant in an engine, a sample of the lubricant has to be removed from the engine for analysis. As a result these methods are generally inconvenient and time-consuming and, in particular, they are not suitable for providing data to an engine management chip.

Methods of identifying lubricants whilst in the engine have been developed but identification is based on relatively crude information such as the viscosity of the oil. In US-A-5274335, an on-board sensor comprising two spaced apart electrodes, a triangular waveform means, a comparison means and a signal means gives an indication of the metallic content of the oil in order to distinguish between two- and four-stroke oils. The information that can be provided about the oil in prior art on-board measurements is limited and, e.g., cannot be used to distinguish between different brands of lubricating oil.

There remains a need for an improved system for identifying lubricating oils in engines that avoids the problems presented by the above prior methods.

The present invention provides a method according to claim 1.

The passive marker provides information about the identity of lubricant, e.g. a specific lubricating oil (i.e. one characterised by brand name and/or performance specification and/or viscosity grade, etc.) will have a unique marker added to it. It has been found that by purposively adding identifying markers to a lubricating oil, which markers are capable of detection in situ in a machine such as an engine, not only can more accurate and specific information be provided as to the identity of the oil but this information can be advantageously utilised by the machine's electronic control unit or management chip. The lubricating oil , when used in a suitable machine (e.g. an engine), enables the machine management chip to distinguish between brands or grades of lubricating oils and react accordingly e.g., by informing the user and/or adjusting machine operation accordingly. This lubricating oil may also be used to ensure that fraudulent lubricating oils are not used in machines such as engines.

Preferably the passive markers are capable of being detected in situ in machine which is on or running.

By "machine" is meant any mechanical equipment having a lubricating system and includes industrial machines and engines, for example automotive engines.

The markers are "passive" in that, in operation, they do not substantially interfere with the performance of the lubricating oil, they do not contribute to the lubricating function of the oil and they do not form a standard component of the oil.

Passive markers suitable for the identification system used in the present invention are odourant molecules.

In the invention, the markers are of a size such that they will pass through an oil filter in the machine (e.g. an engine) in which the oil is intended for use. The marker is an odourant molecule capable of detection by an electronic nose. The choice of odourant molecule is not limited in the present invention. However, preferably it is chosen from compounds such as those known by the trade designations "Ralley", "Blue Spirit", "Lemon Top" or "Petrolica" (all manufactured by Symrise (previously known as Dragoco)). The odourant molecule preferably is present in the lubricating oil composition in an amount of 0.01 to 0.5 % by volume.

In an aspect not part of the invention, the passive markers are of a size such that they will not pass through an oil filter in the machine (e.g. an engine) in which the oil is intended for use. In this aspect not part of the invention, preferably the markers are dimensioned so that they will not pass through filter mesh sizes of between 5 to 50 microns. The concentration of markers in the lubricating oil is such that they will not substantially interfere with the operation of the oil filter. Preferably the lubricating oil comprises from 1 to 10 passive markers of the above type per 4 litres of lubricating oil.

In operation, the markers are collected in the oil filter where they are detected by a detector. The captured markers can then be removed from the oil filter at the next oil change. They may then be recycled or discarded.

Incorporation of the passive markers into the lubricating oil may be done, for example, at the lubricant blending plant.

In another aspect not part of the present invention there is provided the use of an odourant molecule as a passive marker for a lubricating oil.

In another aspect not part of the present invention there is provided a machine such as an engine comprising a detector for detecting in situ a passive marker in the above-described lubricating oil compositions.

A suitable detector for an odourant molecule is an electronic "nose", e.g. a surface acoustic wave electronic nose. Another suitable electronic nose is one based on a sensor array which detects vapours. For example, the sensor array utilised in the device commercially available under the trade designation "Cyranose 320 Electronic Nose" ex. CYRANO Sciences Incorporated adapted for use inside an engine or machine would be suitable (see Examples). For a given lubricant a sensor array gives a unique smell print. The sensor array will be able to detect whether or not the lubricating oil contains a unique odourant and furthermore will also be able to detect whether or not the oil is fresh or partially oxidised.

Preferably the machine (e.g. an engine) comprises an electronic control unit and signals can be transmitted from the electronic nose to said electronic control unit. This may be done either electrically by a wire or by radio frequency techniques at an appropriate frequency including that of 2.45 GHz corresponding to Bluetooth technology. Preferably the machine comprises means to transmit a signal from the electronic nose to the electronic control unit.

In operation, a lubricating oil is put into a machine (e.g. an engine) containing the electronic nose. The electronic nose in the machine will detect whether or not a passive marker is present. The electronic nose accordingly passes a signal to the machine management chip in the machine's electronic control unit. The machine management chip will either recognise the lubricating oil as an oil approved for the machine or as an oil not approved for the machine. In the latter case, this may be as a result of no marker being present. The machine management chip will process this information accordingly. For example, if the lubricant is not approved for use in that machine, then a warning signal may be given to the, e.g., vehicle user and the machine management chip could flag the use of non-approved lubricant to the servicing garage the next time the vehicle has a maintenance check. If the lubricant is approved for use, the machine management chip will adjust the time period until the next oil change in accordance with that particular oil's specification. The machine management chip may process the received information by adjusting the oil change interval prediction algorithm.

Accordingly, the machine comprises an electronic nose and further at least one sensor which indicates the state of the lubricating oil in the machine.

Preferably the sensor measures a parameter which varies with the rate of degradation of the oil in the lubricant system, for example the sensor may provide a measurement as to how oxidised the oil is. The sensor may measure one or more of the following properties of the oil: viscosity, oil level, temperature, pressure, alkalinity/acidity, dielectric constant, capacitance, conductivity and specific density.

In a preferred embodiment, the machine further comprises a pH sensor which gives a reading that is correlated to the Total Acid Number (TAN) of the oil. When the antioxidant of the lubricant is depleted the TAN will start to rise rapidly, indicating that the oil should be changed. In this aspect of the invention, information about the state of the oil may then be used, along with information about the identity of the oil, by the user or the machine management system, to determine when the next oil change is due. This method provides a more accurate determination of when the next oil change is due than those of the prior art. This is because not only does it take into account the specification of the particular oil, it also takes into account the actual state of the oil.

For example, with regard to engines, the period between oil changes as recommended by the OEM is frequently based on time and/or distance travelled (e.g. frequently a handbook may say that the oil needs changing every year or 9000 miles whichever occurs first). However, this does not take into account the effect different types of engine use have on the lubricant. High speed, long motorway journeys typically put less "stress" on the oil than frequent cold starts and short trip journeys. By utilising a measurement of the state of the oil to assess when an oil change is necessary, the effect that driving conditions has had on the oil is inherently taken into account.

The electronic nose may also advantageously act as a sensor which indicates the state of the lubricating oil in the engine or machine. The smell of oil changes as oil ages and this change may be used as an indication of the state of the oil and therefore as an indication of when the next oil change is due.

Preferably the machine comprises means to transmit a signal from at the least one sensor to the machine's electronic control unit or the machine management chip.

In operation, as the machine further comprises a sensor for measuring the state of the oil, this sensor will also pass a signal to the machine management chip. The machine management chip will process the information accordingly, e.g. by using the information along with that received about the identity of the oil to determine when the next oil change is required or to set values for determining when a next oil change is required.

Accordingly the present invention provides a method according to claim 1.

A display may be coupled to machine management system or electronic control unit and may indicate to the user the type of oil present and when the next oil change is due.

In a preferred embodiment of the present invention, the afore-mentioned machine is an engine.

According to another aspect not part of the present invention there is provided a vehicle comprising an engine as described above and a method of operating said vehicle.

According to another aspect also not part of the present invention there is provided a lubricating oil identification system comprising:
(i) a lubricating oil composition as described above; and
(ii) a detector for detecting a passive marker in said oil composition when the oil composition is in the machine.

The identification system may further comprise a machine (e.g. an engine) as described above.

The present invention will be described in greater detail with reference to the following examples. The present invention is, however, not limited thereto.

### Example 1

This example demonstrates how an electronic nose can be trained to distinguish between passenger car motor oil samples that are fresh, heavily oxidised and contain an odourant marker.

The electronic nose used in this example is a commercially available electronic "nose" available under the trade designation "Cyranose 320 Electronic Nose", manufactured by CYRANO Sciences Incorporated. The particular model used was a multimeter design and as such is not suitable for use in an engine. However, the sensor array that actually detects the vapours is a small chip which may be adapted for use in an engine. The chip comprises 32 individual thin-film carbon-black polymer composite chemiresistors configured into an array. The sensor materials are thin films deposited across two electrical leads on an alumina substrate, creating the conducting chemiresistors. When the composite film is exposed to a vapour-phase analyte, the polymer matrix acts like a sponge and swells while absorbing the analyte. The increase in volume causes an increase in resistance because the conductive carbon-black pathways through the material are disrupted. When the analyte is removed the polymer releases the analyte and shrinks to its original size, restoring the conductive pathways. Each polymer used in the array is chemically unique and absorbs the analyte gases to a different degree. This creates a pattern of differential response across the array. For a given lubricant, the sensor array gives a unique "smell print".

Fresh oil samples were obtained from typical gasoline and diesel engine oil formulations of differing viscosity grades, base oil types and additive packages obtained from major lubricant manufacturers.

In order to test how the electronic nose can be trained to distinguish between passenger car motor oil samples that are fresh and heavily oxidised, heavily oxidised oil samples were prepared by the method disclosed at the 13^{th} International Colloquium Tribology, Esslingen, 15-17 January 2002 by Shell Global Solutions (UK), which comprises taking fresh oil samples and using a Shell proprietary laboratory blown air oxidation rig in which the oils were kept at 150°C and NOₓ in air was bubbled through the oil at a prescribed rate so that oxidation conditions were similar to those of the ASTM industry standard Sequence IIIE engine test. In addition, a small amount of metal catalysts were added to the lubricating oils to simulate typical wear metal concentrations found in engine oil sumps.

The samples were subjected to these conditions for differing periods of time, due to the fact that the oils oxidised at different rates. In the laboratory test, the Total Base Number (TBN), the Total Acid Number (TAN), and the viscosity increase was monitored in order that it could be determined if the lubricant was heavily oxidised or not.

This oxidised sample preparation was carried out by utilising the publicly available commercial oxidised sample preparation service that is advertised and available ex. Shell Global Solutions (UK), Cheshire Innovation Park, P.O. Box 1, Chester CH1 3SH, UK.
(Email: shellglobalsolutions@OPC.shell.com).

Oil samples containing odourant were prepared by incorporating the odourant available under the trade designation "Lemon Top" or the odourant available under the trade designation "Ralley" (ex. Symrise (previously known as Dragoco)) into the lubricating oil.

These oil samples were tested using the afore-mentioned "Cyranose 320" multimeter. The experiment was set up as follows:
Baseline purge: 30 secs; pump speed: medium
Sample draw: 90 secs; pump speed: medium
Sample draw 2: 0 secs
Snout removal: 0 secs
1^{st} sample gas purge: 0 secs
1^{st} air intake purge: 30 secs; pump speed: high
2^{nd} sample gas purge: 30 secs; pump speed: high
2^{nd} air intake purge: 30 secs; pump speed: high

Digital filtering: On
Substrate heater: On, 37°C
Training repeat count: 1
Identifying repeat count: 1

Active sensors: 05, 06, 23 and 31 switched off, all others were on.

Algorithm: Canonical
Preprocessing: Auto-scaling

Normalisation: 1
Identification quality: Higher

The samples were prepared for testing as follows: Approximately 4 ml of sample was transferred using a clean plastic pastette into a 40 ml clean glass vial and sealed with PTFE-butyl septum. The samples were then left in a 50°C oven for one-and-a-half hours to allow the vapours to equilibrate in the headspace. A G15 luer mount 9 cm stainless steel hypodermic needle was attached to the nose to pierce the septum and draw the sample. Another needle was used to vent the vial to ensure that the pressure was equalised during the sample draw.

### Example 2

The "Cyranose C320" multimeter was "trained" on ten different samples of gasoline engine oils and their respective completely oxidised forms. The training samples were tested at random to make the training set as versatile as possible. The canonical data for the training set is shown below in Table 1. All the fresh oil data lies in the range of 2.8 to 5.9 for Factor 1, whereas all the oxidised oil data lies in the range of - 5.25 to -2.2 for Factor 1. The two data sets are well separated and it is clear that the fresh oils and heavily oxidised oils are easily distinguishable.

Once the training set was validated using the "cross-validation" option available on the "Cyranose C320" multimeter, six "unknown" samples (A-F) were tested at random to check that the "Cyranose C320" multimeter could identify them. All of the unknown samples tested were correctly identified as being fresh or completely oxidised.

Sensors 05, 06, 23 and 31 were switched off since earlier investigations had identified that they gave erratic readings during the first 10 to 20 seconds of the sample draw (it is thought this is due to the possible presence of water vapour).

**Table 1**

| Sample No. | Sample Details | Factor 1 | Factor 2 |
|---|---|---|---|
| 1 | Fresh Shell "Helix" Plus SAE-10W/40 | 2.8 | 0 |
| 2 | Fresh Shell "Helix" Plus SAE-10W/40 | 5.9 | 0 |
| 3 | Fresh Shell "Helix" Plus SAE-10W/40 | 3.6 | 0 |
| 4 | Fresh Shell "Helix" Plus SAE-10W/40 | 3.8 | 0 |
| 5 | Fresh Shell "Helix" Plus SAE-10W/40 | 3.7 | 0 |
| 6 | Fresh Shell "Helix" Plus SAE-10W/40 | 5.2 | 0 |
| 7 | Fresh Shell "Helix" Plus SAE-10W/40 | 3.8 | 0 |
| 8 | Fresh Shell "Helix" Plus SAE-10W/40 | 4 | 0 |
| 9 | Fresh Shell "Helix" Plus SAE-10W/40 | 4.1 | 0 |
| 10 | Fresh Shell "Helix" Plus SAE-10W/40 | 3 | 0 |
| 1a | Fresh Shell "Helix" Plus SAE-10W/40 oxidised for 196 hours in rig | -5.25 | 0 |
| 2a | Fresh Shell "Helix" Plus SAE-10W/40 oxidised for 196 hours in rig | -5.2 | 0 |
| 3a | Fresh Shell "Helix" Plus SAE-10W/40 oxidised for 196 hours in rig | -3.2 | 0 |
| 4a | Fresh Shell "Helix" Plus SAE-10W/40 oxidised for 196 hours in rig | -4.3 | 0 |
| 5a | Fresh Shell "Helix" Plus SAE-10W/40 oxidised for 196 hours in rig | -3.1 | 0 |
| 6a | Fresh Shell "Helix" Plus SAE-10W/40 oxidised for 196 hours in rig | -4.4 | 0 |
| 7a | Fresh Shell "Helix" Plus SAE-10W/40 oxidised for 196 hours in rig | -2.2 | 0 |
| 8a | Fresh Shell "Helix" Plus SAE-10W/40 oxidised for 196 hours in rig | -4.5 | 0 |
| 9a | Fresh Shell "Helix" Plus SAE-10W/40 oxidised for 196 hours in rig | -4.3 | 0 |
| 10a | Fresh Shell "Helix" Plus SAE-10W/40 oxidised for 196 hours in rig | -2.8 | 0 |

| | | | |
|---|---|---|---|
| N.B. "HELIX" is a Shell trade mark. | | | |

### Example 3

A second set of experiments was carried out which included fresh oils containing odourant molecules. The canonical plot for this experiment is shown in Table 2. The fresh oil data lies in the range of -3.3 to -8.5 for Factor 1 and in the range 0.75 to 4.0 for Factor 2. The oxidised oil data lies in the range of -5.6 to -6.1 for Factor 1 and the range -0.9 to -3 for Factor 2. The fresh oil with the added odourant lies in the range of 7 to 10.2 for Factor 1 and -1.8 to 0.1 for Factor 2. Again the three different data sets are well separated.

Table 2 demonstrates that the sensor contained in the "Cyranose C320 Electronic Nose" can distinguish fresh lubricant from degraded lubricant, and can also distinguish fresh lubricant from fresh lubricant containing an odourant.

**Table 2**

| Sample No. | Sample Details | Factor 1 | Factor 2 |
|---|---|---|---|
| 1 | Fresh Shell "Helix" Plus SAE-10W/40 | -3.3 | 3.7 |
| 2 | Fresh Shell "Helix" Plus SAE-10W/40 | -1.8 | 3 |
| 3 | Fresh Shell "Helix" Plus SAE-10W/40 | -3 | 2.2 |
| 4 | Fresh Shell "Helix" Plus SAE-10W/40 | -0.85 | 4 |
| 5 | Fresh Shell "Helix" Plus SAE-10W/40 | -1.7 | 0.75 |
| 1a | Fresh Shell "Helix" Plus SAE-10W/40 oxidised for 196 hours in rig | -6 | -2.1 |
| 2a | Fresh Shell "Helix" Plus SAE-10W/40 oxidised for 196 hours in rig | -6.1 | -3 |
| 3a | Fresh Shell "Helix" Plus SAE-10W/40 oxidised for 196 hours in rig | -6 | -0.9 |
| 4a | Fresh Shell "Helix" Plus SAE-10W/40 oxidised for 196 hours in rig | -5.8 | -2.3 |
| 5a | Fresh Shell "Helix" Plus SAE-10W/40 oxidised for 196 hours in rig | -5.6 | -1.8 |
| 1b | Fresh Shell "Helix" Plus SAE-10W/40 with 0.25 % by volume Symrise "Lemon Top" odourant added | 10.2 | -0.2 |
| 2b | Fresh Shell "Helix" Plus SAE-10W/40 with 0.25 % by volume Symrise "Lemon Top" odourant added | 7.25 | -0.8 |
| 3b | Fresh Shell "Helix" Plus SAE-10W/40 with 0.25 % by volume Symrise "Lemon Top" odourant added | 7 | -1.1 |
| 4b | Fresh Shell "Helix" Plus SAE-10W/40 with 0.25 % by volume Symrise "Lemon Top" odourant added | 7.25 | 0.1 |
| 5b | Fresh Shell "Helix" Plus SAE-10W/40 with 0.25 % by volume Symrise "Lemon Top" odourant added | 9.2 | -1.8 |

Table 3 shows the data for "smell print" for each of the three types of oil (fresh, fresh + odourant, heavily oxidised). Each of the three types of oil has a unique "smell print" which can be recognised by the electronic nose. The data for each type of oil is the maximum change in sensory output (arbitrary units) for each sensor. Sensors 5, 6, 23 and 31 were switched off in these experiments.

This example demonstrates how specific odourants can be utilised as markers for oil and an electronic nose that utilises a sensor array of the same type that is found in the "Cyranose 320" Electronic Nose can be utilised as a detector in the identification system used in the present invention. The output from the sensor would be (1) fresh oil + odourant (i.e. the specific type of oil could be identified), (2) fresh oil without an odourant, or (3) heavily oxidised oil. This output can be sent to the electronic management system of the vehicle. If the signal (1) was received, the engine management system can check whether the specific type of lubricant identified was one that was approved by OEM. If signal (2) was received, the engine management system can warn the user of the vehicle that they should use an OEM approved lubricant. If signal (3) is received by the engine management system, a light or other warning mechanism can be used to advise the user that the lubricant requires changing.

**Table 3**

| Sensor in Array | Fresh Oil: Shell "Helix" Plus SAE-10W/40 (Sensor output in arbitrary units) | Oxidised Oil: Shell "Helix" Plus SAE-10W/40 oxidised for 196 hours (Sensor output in arbitrary units) | oil with Odourant: Shell "Helix" Plus SAE-10W/40 with 0.03 % by volume of Symrise "Ralley" Odourant (Sensor output in arbitrary units) |
|---|---|---|---|
| 1 | 4072.5 | 36907.6 | 16344.0 |
| 2 | 4601.7 | 44224.9 | 17397.3 |
| 3 | 4009.7 | 42512.5 | 16033.3 |
| 4 | 2400.5 | 25036.7 | 10661.4 |
| 5 | | | |
| 6 | | | |
| 7 | 833.3 | 6342.7 | 2179.6 |
| 8 | 882.8 | 10033.1 | 5115.1 |
| 9 | 2501.0 | 33780.8 | 12644.7 |
| 10 | 177.6 | 1365.1 | 434.2 |
| 11 | 997.1 | 6014.3 | 2003.8 |
| 12 | 2849.2 | 40477.9 | 16134.7 |
| 13 | 560.0 | 5649.4 | 2331.8 |
| 14 | 1039.5 | 14060.4 | 7235.9 |
| 15 | 569.7 | 12273.2 | 2656.8 |
| 16 | 363.2 | 6736.5 | 2249.2 |
| 17 | 94.7 | 3085.0 | 743.1 |
| 18 | 1651.7 | 21180.4 | 11110.2 |
| 19 | 349.0 | 3721.5 | 1906.3 |
| 20 | 1771.7 | 35433.7 | 9891.2 |
| 21 | 192.9 | 3386.5 | 1150.3 |
| 22 | 288.6 | 1280.5 | 664.2 |
| 23 | | | |
| 24 | 539.5 | 4161.1 | 2539.8 |
| 25 | 754.3 | 9665.9 | 2723.4 |
| 26 | 1628.3 | 18503.2 | 6398.5 |
| 27 | 1527.0 | 17988.9 | 14194.6 |
| 28 | 2859.1 | 31129.1 | 12856.2 |
| 29 | 4088.5 | 48187.4 | 17863.9 |
| 30 | 1114.9 | 9784.8 | 3703.6 |
| 31 | | | |
| 32 | 1050.9 | 8934.6 | 5206.3 |

## Claims

1. A method to determine when an oil change is required in a machine, said machine comprising an electronic nose for detecting in situ a passive marker in a lubricating oil when the lubricating oil is in the machine, wherein the passive marker is an odourant molecule; and at least one sensor which indicates the state of the oil in the machine, said method comprising:
(i) using the electronic nose to provide data about the identity of the lubricating oil in the machine;
(ii) using the at least one sensor to provide data indicating the state of the oil; and
(iii) utilising the data obtained in (i) and (ii) to determine when an oil change is required or to set values which can be used to determine when an oil change is required.

2. Method according to Claim 1, wherein the machine comprises an electronic control unit and means to transmit a signal from the electronic nose to the electronic control unit.

3. Method according to Claim 1 or 2, wherein the machine comprises an electronic control unit and means to transmit a signal from the at least one sensor to the electronic control unit.

4. Method according to any one of Claims 1 to 3,
wherein the machine is mechanical equipment having a lubricating system.

5. Method according to any one of Claims 1 to 4,
wherein the machine is an engine.

6. Method according.to any one of Claims 1 to 5,
wherein the odourant molecule is present in the lubricating oil in an amount of 0.01 to 0.5 % by volume.

## Patentansprüche

1. Verfahren zum Bestimmen, wann ein Ölwechsel in einer Maschine erforderlich ist, wobei die Maschine eine elektronische Nase zum Ermitteln eines passiven in situ-Markers in einem Schmieröl aufweist, wenn sich das Schmieröl in der Maschine befindet, wobei der passive Marker ein Geruchsmolekül ist; und zumindest einen Sensor, der den Status des Öls in der Maschine anzeigt, wobei das Verfahren umfaßt:
(i) Verwendung der elektronischen Nase zum Liefern von Daten über die Identität des Schmieröls in der Maschine;
(ii) Verwendung des zumindest einen Sensors zum Liefern von Daten, welche den Status des Öls anzeigen; und
(iii) Verwendung der in (i) und (ii) erhaltenen Daten zum Bestimmen, wann ein Ölwechsel erforderlich ist, oder zum Einstellen von Werten, die verwendet werden können, um zu bestimmen, wann ein Ölwechsel erforderlich ist.

2. Verfahren nach Anspruch 1, bei welchem die Maschine eine elektronische Steuereinrichtung und Mittel zum Übertragen eines Signals von der elektronischen Nase zur elektronischen Steuereinheit aufweist.

3. Verfahren nach Anspruch 1 oder 2, bei welchem die Maschine eine elektronische Steuereinrichtung und Mittel zum Übertragen eines Signals von dem zumindest einen Sensor zur elektronischen Steuereinrichtung aufweist.

4. Verfahren nach einem der Ansprüche 1 bis 3, bei welchem die Maschine eine mechanische Ausrüstung ist, die ein Schmiersystem aufweist.

5. Verfahren nach einem der Ansprüche 1 bis 4, bei welchem die Maschine ein Motor ist.

6. Verfahren nach einem der Ansprüche 1 bis 5, bei welchem das Geruchsmolekül in dem Schmieröl in einer Menge von 0,01 bis 0,5 Vol.-% vorhanden ist.

## Revendications

1. Procédé de détermination du moment où une vidange d'huile est requise dans une machine, ladite machine comprenant un nez électronique pour détecter in situ un révélateur passif dans une huile quand l'huile lubrifiante est dans la machine, dans lequel le révélateur passif est une molécule odorante ; et au moins un capteur qui indique l'état de l'huile dans la machine, ledit procédé comprenant les étapes consistant à :
(i) utiliser le nez électronique pour fournir des données concernant l'identité de l'huile lubrifiante dans la machine ;
(ii) utiliser l'au moins un capteur pour fournir des données indiquant l'état de l'huile ; et
(iii) utiliser les données obtenues aux étapes (i) et (ii) pour déterminer le moment où une vidange d'huile est requise ou pour établir des valeurs qui puissent être utilisées pour déterminer le moment où une vidange d'huile est requise.

2. Procédé selon la revendication 1, dans lequel la machine comprend une unité de contrôle électronique et des moyens de transmission d'un signal issu du nez électronique à l'unité de contrôle électronique.

3. Procédé selon la revendication 1 ou 2, dans lequel la machine comprend une unité de contrôle électronique et des moyens de transmission d'un signal issu de l'au moins un capteur à l'unité de contrôle électronique.

4. Procédé selon l'une quelconque des revendications 1 à 3, dans lequel la machine est un équipement mécanique muni d'un système de lubrification.

5. Procédé selon l'une quelconque des revendications 1 à 4, dans lequel la machine est un moteur.

6. Procédé selon l'une quelconque des revendications 1 à 5, dans lequel la molécule odorante est présente dans l'huile lubrifiante dans une proportion de 0,01 à 0,5% en volume.
